# EUROPEAN PATENT APPLICATION

(11) **EP 3 871 679 A1**
(43) Date of publication of application: **01.09.2021**
(21) Application number: 19875072.1
(22) Date of filing: 25.10.2019
(51) Int. Cl.: A61K 31/7088, A61P 43/00, C12N 15/113

(54) **METHOD FOR SUPPRESSING PROTEIN TRANSLATION REACTION USING STAPLE NUCLEIC ACID**

(30) Priority: 25.10.2018 JP 2018201074
(71) Applicant: National University Corporation Kumamoto University, Kumamoto-shi, Kumamoto 860-8555 (JP); HIROSAKI UNIVERSITY, Hirosaki-shi, Aomori 036-8560 (JP)
(72) Inventor: KATSUDA Yosuke, Kumamoto-shi, Kumamoto 860-8555 (JP); SATO Shinichi, Kyoto-shi, Kyoto 606-8501 (JP); HAGIHARA Masaki, Hirosaki-shi, Aomori 036-8560 (JP); IHARA Toshihiro, Kumamoto-shi, Kumamoto 860-8555 (JP); KITAMURA Yusuke, Kumamoto-shi, Kumamoto 860-8555 (JP)
(74) Representative: J A Kemp LLP
(86) International application number: PCT/JP2019/042049
(87) International publication number: WO 2020/085510

(57) **Abstract**

There is provided a method for suppressing protein expression, including using an oligomer for RNA having first, second, third, and fourth guanine repeat sequences (referred to as "first G sequence," "second G sequence," "third G sequence," and "fourth G sequence," respectively), the oligomer hybridizing with regions proximal to the guanine repeat sequences, so as to bring the guanine repeat sequences closer together, thereby forming a guanine-quadruplex structure.

Sequence Listing

SEQUENCE LISTING

<110>  Kumamoto University
       Hirosaki University

<120>   A method of inhibiting protein translation using a Staple
       nucleic acid

<130>  G2153WO

<150>  JP2018-201074
<151>  2018-10-25

<160>  90

<170>  PatentIn version 3.5

<210>  1
<211>  44
<212>  DNA
<213>  Artificial

<220>
<223>  Synthetic DNA

<400>  1
taatacgact cactatagat tagcatacgc tactgcagat gcgc                        44


<210>  2
<211>  44
<212>  DNA
<213>  Artificial

<220>
<223>  Synthetic DNA

<400>  2
gctgaatagc ttgcaagtca tggcatgcgc atctgcagta gcgt                        44


<210>  3
<211>  18
<212>  DNA
<213>  Artificial

<220>
<223>  Synthetic DNA

<400>  3
taatacgact cactatag                                                     18


<210>  4
<211>  36
<212>  DNA
<213>  Artificial

<220>
<223>  Synthetic DNA

<400>  4
tccaactatg tatacctgct gaatagcttg caagtc                                 36

<210>  5
<211>  35
<212>  DNA
<213>  Artificial

<220>
<223>  Synthetic DNA

<400>  5
ctatagtgag tcgtattaaa tcgtcgaacg gcagg                                35


<210>  6
<211>  35
<212>  DNA
<213>  Artificial

<220>
<223>  Synthetic DNA

<400>  6
caggtataca tagttggaaa tctctggaag atccg                                35


<210>  7
<211>  312
<212>  DNA
<213>  Artificial

<220>
<223>  Synthetic DNA

<400>  7
acacaggaaa cagctatgac catgattacg ccaagtttgc acgcctgccg ttcgacgatt      60

taatacgact cactatagat tagcatacgc tactgcagtg ggtgggtgtc gacctagatt     120

aatgcaattc gtacgaagtt catagcattt ccagcaccca attgaagctt tgggtgggtg     180

catgccatga cttgcaagct attcagcagg tatacatagt tggaaatctc tggaagatcc     240

gcgcgtaccg agttctaatt cactggccgt cgttttacaa cgtcgtgact gggaaaaccc     300

tggcgttacc ca                                                         312


<210>  8
<211>  20
<212>  DNA
<213>  Artificial

<220>
<223>  Synthetic DNA

<400>  8
taatacgact cactatagaa                                                 20


<210>  9
<211>  18
<212>  DNA

<213>  Artificial

<220>
<223>  Synthetic DNA

<400>  9
tccaactatg tatacctg                                                    18


<210>  10
<211>  49
<212>  DNA
<213>  Artificial

<220>
<223>  Synthetic DNA

<400>  10
cctagattaa tgcaattcgt gtgaagatcc tttttgataa tctcatgac                  49


<210>  11
<211>  49
<212>  DNA
<213>  Artificial

<220>
<223>  Synthetic DNA

<400>  11
tcaattgggt gctggaaatg aactcacgtt aaggattttt ggtcatgag                  49


<210>  12
<211>  25
<212>  DNA
<213>  Artificial

<220>
<223>  Synthetic DNA

<400>  12
catttccagc acccaattga agctt                                            25


<210>  13
<211>  25
<212>  DNA
<213>  Artificial

<220>
<223>  Synthetic DNA

<400>  13
acgaattgca ttaatctagg tcgac                                            25


<210>  14
<211>  34
<212>  DNA
<213>  Artificial

<220>

<223>  Synthetic DNA

<400>  14
ctagcctcga gaattgatta gcatacgcta ctgc                                    34


<210>  15
<211>  35
<212>  DNA
<213>  Artificial

<220>
<223>  Synthetic DNA

<400>  15
ccatggtggc gaattctgaa tagcttgcaa gtcat                                   35


<210>  16
<211>  43
<212>  DNA
<213>  Artificial

<220>
<223>  Synthetic DNA

<400>  16
agggattagc atacgctact gcagtaaata aatgtcgacc tag                          43


<210>  17
<211>  41
<212>  DNA
<213>  Artificial

<220>
<223>  Synthetic DNA

<400>  17
cggtacccgg ggatctaata cgactcacta tagggattag c                            41


<210>  18
<211>  26
<212>  DNA
<213>  Artificial

<220>
<223>  Synthetic DNA

<400>  18
cacgaattgc attaatctag gtcgac                                             26


<210>  19
<211>  26
<212>  DNA
<213>  Artificial

<220>
<223>  Synthetic DNA

<400>  19

gtcgacctag attaatgcaa ttcgtg 26

<210> 20
<211> 25
<212> DNA
<213> Artificial

<220>
<223> Synthetic DNA

<400> 20
aagcttcaat tgggtgctgg aaatg 25

<210> 21
<211> 38
<212> DNA
<213> Artificial

<220>
<223> Synthetic DNA

<400> 21
attgaagctt taaataaatg catgccatga cttgcaag 38

<210> 22
<211> 25
<212> DNA
<213> Artificial

<220>
<223> Synthetic DNA

<400> 22
catttccagc acccaattga agctt 25

<210> 23
<211> 35
<212> DNA
<213> Artificial

<220>
<223> Synthetic DNA

<400> 23
cgactctaga ggatcctgaa tagcttgcaa gtcat 35

<210> 24
<211> 31
<212> DNA
<213> Artificial

<220>
<223> Synthetic DNA

<400> 24
taatacgact cactataggg ctagcctcga g 31

<210>  25
<211>  35
<212>  DNA
<213>  Artificial

<220>
<223>  Synthetic DNA

<400>  25
ccatggtggc gaattctgaa tagcttgcaa gtcat                                35


<210>  26
<211>  20
<212>  DNA
<213>  Artificial

<220>
<223>  Synthetic DNA

<400>  26
taatacgact cactataggg                                                 20


<210>  27
<211>  35
<212>  DNA
<213>  Artificial

<220>
<223>  Synthetic DNA

<400>  27
cgactctaga ggatcctgaa tagcttgcaa gtcat                                35


<210>  28
<211>  25
<212>  DNA
<213>  Artificial

<220>
<223>  Synthetic DNA

<400>  28
gtcacatccg ggcgggttgg tgagt                                           25


<210>  29
<211>  25
<212>  DNA
<213>  Artificial

<220>
<223>  Synthetic DNA

<400>  29
ggtgcccacc cagctactgc tcgcg                                           25


<210>  30
<211>  25
<212>  DNA

<213>  Artificial

<220>
<223>  Synthetic DNA

<400>  30
agattcctac ttcttacgcc cccca                                          25


<210>  31
<211>  25
<212>  DNA
<213>  Artificial

<220>
<223>  Synthetic DNA

<400>  31
tgtctgccag cgcttcctct ttctc                                          25


<210>  32
<211>  43
<212>  DNA
<213>  Artificial

<220>
<223>  Synthetic DNA

<400>  32
taatacgact cactataggt cacatccggg cgggttggtg agt                      43


<210>  33
<211>  43
<212>  DNA
<213>  Artificial

<220>
<223>  Synthetic DNA

<400>  33
tccaactatg tatacctggg tgcccaccca gctactgctc gcg                      43


<210>  34
<211>  43
<212>  DNA
<213>  Artificial

<220>
<223>  Synthetic DNA

<400>  34
taatacgact cactatagag attcctactt cttacgcccc cca                      43


<210>  35
<211>  43
<212>  DNA
<213>  Artificial

<220>

<223>  Synthetic DNA

<400>  35
tccaactatg tatacctgtg tctgccagcg cttcctcttt ctc                43


<210>  36
<211>  35
<212>  DNA
<213>  Artificial

<220>
<223>  Synthetic DNA

<400>  36
acacaggaaa cagctatgac catgattacg ccaag                35


<210>  37
<211>  35
<212>  DNA
<213>  Artificial

<220>
<223>  Synthetic DNA

<400>  37
tgggtaacgc cagggttttc ccagtcacga cgttg                35


<210>  38
<211>  40
<212>  DNA
<213>  Artificial

<220>
<223>  Synthetic DNA

<400>  38
ctagcctcga gaattcgcca ccatggccga tgctaagaac                40


<210>  39
<211>  33
<212>  DNA
<213>  Artificial

<220>
<223>  Synthetic DNA

<400>  39
ctcgacgcgt gaattttaca cggcgatctt gcc                33


<210>  40
<211>  34
<212>  DNA
<213>  Artificial

<220>
<223>  Synthetic DNA

<400>  40

ctagcctcga gaattgatta gcatacgcta ctgc                                    34

<210>  41
<211>  35
<212>  DNA
<213>  Artificial

<220>
<223>  Synthetic DNA

<400>  41
ccatggtggc gaattctgaa tagcttgcaa gtcat                                   35

<210>  42
<211>  33
<212>  DNA
<213>  Artificial

<220>
<223>  Synthetic DNA

<400>  42
ctagcctcga gaatttcaca tccgggcggg ttg                                     33

<210>  43
<211>  35
<212>  DNA
<213>  Artificial

<220>
<223>  Synthetic DNA

<400>  43
ctagcctcga gaattagatt cctacttctt acgcc                                   35

<210>  44
<211>  33
<212>  DNA
<213>  Artificial

<220>
<223>  Synthetic DNA

<400>  44
ccatggtggc gaattgtttt cccagtcacg acg                                     33

<210>  45
<211>  31
<212>  DNA
<213>  Artificial

<220>
<223>  Synthetic DNA

<400>  45
taatacgact cactataggg ctagcctcga g                                       31

<210>  46
<211>  31
<212>  DNA
<213>  Artificial

<220>
<223>  Synthetic DNA

<400>  46
taatacgact cactataggg tcacatccgg g                                    31


<210>  47
<211>  30
<212>  DNA
<213>  Artificial

<220>
<223>  Synthetic DNA

<400>  47
taatacgact cactataggg agattcctac                                      30


<210>  48
<211>  33
<212>  DNA
<213>  Artificial

<220>
<223>  Synthetic DNA

<400>  48
ctcgacgcgt gaattttaca cggcgatctt gcc                                  33


<210>  49
<211>  41
<212>  DNA
<213>  Artificial

<220>
<223>  Synthetic DNA

<400>  49
ctagcctcga gaattcgcca ccatggtctt cacactcgaa g                         41


<210>  50
<211>  41
<212>  DNA
<213>  Artificial

<220>
<223>  Synthetic DNA

<400>  50
ctagcctcga gaattcgcca ccatggtctt cacactcgaa g                         41


<210>  51
<211>  34
<212>  DNA

<213>  Artificial

<220>
<223>  Synthetic DNA

<400>  51
tcgacccggg cggccatggc ttccaaggtg tacg                                    34


<210>  52
<211>  34
<212>  DNA
<213>  Artificial

<220>
<223>  Synthetic DNA

<400>  52
taaagggaag cggccttact gctcgttctt cagc                                    34


<210>  53
<211>  33
<212>  DNA
<213>  Artificial

<220>
<223>  Synthetic DNA

<400>  53
ctagcctcga gaatttcaca tccgggcggg ttg                                     33


<210>  54
<211>  35
<212>  DNA
<213>  Artificial

<220>
<223>  Synthetic DNA

<400>  54
ctagcctcga gaattagatt cctacttctt acgcc                                   35


<210>  55
<211>  33
<212>  DNA
<213>  Artificial

<220>
<223>  Synthetic DNA

<400>  55
ccatggtggc gaattgtttt cccagtcacg acg                                     33


<210>  56
<211>  43
<212>  DNA
<213>  Artificial

<220>

<223>  Synthetic DNA

<400>  56
gatcccctac cggaactcac cagctactgc tcgcgctttt tta                43


<210>  57
<211>  43
<212>  DNA
<213>  Artificial

<220>
<223>  Synthetic DNA

<400>  57
agcttaaaaa agcgcgagca gtagctggtg agttccggta ggg                43


<210>  58
<211>  39
<212>  DNA
<213>  Artificial

<220>
<223>  Synthetic DNA

<400>  58
gatccccccg gaactcacca gctactgctc gcgtttttta                    39


<210>  59
<211>  39
<212>  DNA
<213>  Artificial

<220>
<223>  Synthetic DNA

<400>  59
agcttaaaaa cgcgagcagt agctggtgag ttccggggg                     39


<210>  60
<211>  43
<212>  DNA
<213>  Artificial

<220>
<223>  Synthetic DNA

<400>  60
gatcccctct atctcctgcg gcacaatctg gagccccttt tta                43


<210>  61
<211>  43
<212>  DNA
<213>  Artificial

<220>
<223>  Synthetic DNA

<400>  61

agcttaaaaa ggggctccag attgtgccgc aggagataga ggg 43

<210> 62
<211> 39
<212> DNA
<213> Artificial

<220>
<223> Synthetic DNA

<400> 62
   gatcccctat ctcctgcggc acaatctgga gccttttta 39

<210> 63
<211> 39
<212> DNA
<213> Artificial

<220>
<223> Synthetic DNA

<400> 63
agcttaaaaa ggctccagat tgtgccgcag gagataggg 39

<210> 64
<211> 40
<212> DNA
<213> Artificial

<220>
<223> Synthetic DNA

<400> 64
ttgcattaat ctaggtcgac aagcttcaat tgggtgctgg 40

<210> 65
<211> 40
<212> DNA
<213> Artificial

<220>
<223> Synthetic DNA

<400> 65
ttgcattaat ctaggtcgac aagcttcaat tgggtgctgg 40

<210> 66
<211> 40
<212> DNA
<213> Artificial

<220>
<223> Synthetic DNA

<400> 66
aagcttcaat tgggtgctgg ttgcattaat ctaggtcgac 40

<210>  67
<211>  40
<212>  DNA
<213>  Artificial

<220>
<223>  Synthetic DNA

<400>  67
gaaataccgg aactcaccaa agctactgct cgcgctccgg                              40


<210>  68
<211>  40
<212>  DNA
<213>  Artificial

<220>
<223>  Synthetic DNA

<400>  68
ttcctctttc tcctgcggca tacaatctgg agccccgagc                              40


<210>  69
<211>  30
<212>  RNA
<213>  Artificial

<220>
<223>  Synthetic RNA

<400>  69
uuaaucuagg ucgacaagcu ucaauugggu                                         30


<210>  70
<211>  26
<212>  RNA
<213>  Artificial

<220>
<223>  Synthetic RNA

<400>  70
aaucuagguc gacaagcuuc aauugg                                             26


<210>  71
<211>  40
<212>  DNA
<213>  Artificial

<220>
<223>  Synthetic DNA

<400>  71
ttgcattaat ctaggtcgac aagcttcaat tgggtgctgg                              40


<210>  72
<211>  40
<212>  DNA

<213>  Artificial

<220>
<223>  Synthetic DNA

<400>  72
aagcttcaat tgggtgctgg ttgcattaat ctaggtcgac                              40


<210>  73
<211>  40
<212>  DNA
<213>  Artificial

<220>
<223>  Synthetic DNA

<400>  73
tgaaataccg gaactcacca gctactgctc gcgctccggt                              40


<210>  74
<211>  40
<212>  DNA
<213>  Artificial

<220>
<223>  Synthetic DNA

<400>  74
cttcctcttt ctcctgcggc acaatctgga gccccgagca                              40


<210>  75
<211>  30
<212>  RNA
<213>  Artificial

<220>
<223>  Synthetic RNA

<400>  75
uaccggaacu caccagcuac ugcucgcgcu                                         30


<210>  76
<211>  26
<212>  RNA
<213>  Artificial

<220>
<223>  Synthetic RNA

<400>  76
ccggaacuca ccagcuacug cucgcg                                             26


<210>  77
<211>  30
<212>  RNA
<213>  Artificial

<220>

<223>  Synthetic RNA

<400>  77
ucuaucuccu gcggcacaau cuggagcccc                                        30


<210>  78
<211>  26
<212>  RNA
<213>  Artificial

<220>
<223>  Synthetic RNA

<400>  78
uaucuccugc ggcacaaucu ggagcc                                            26


<210>  79
<211>  41
<212>  DNA
<213>  Artificial

<220>
<223>  Synthetic DNA

<400>  79
ctagcctcga gaattcgcca ccatggtctt cacactcgaa g                           41


<210>  80
<211>  34
<212>  DNA
<213>  Artificial

<220>
<223>  Synthetic DNA

<400>  80
ctcgacgcgt gaattttacg ccagaatgcg ttcg                                   34


<210>  81
<211>  34
<212>  DNA
<213>  Artificial

<220>
<223>  Synthetic DNA

<400>  81
tcgacccggg cggccatggc ttccaaggtg tacg                                   34


<210>  82
<211>  34
<212>  DNA
<213>  Artificial

<220>
<223>  Synthetic DNA

<400>  82

taaagggaag cggccttact gctcgttctt cagc                                    34

<210>  83
<211>  25
<212>  DNA
<213>  Artificial

<220>
<223>  Synthetic DNA

<400>  83
gtcacatccg ggcgggttgg tgagt                                             25

<210>  84
<211>  25
<212>  DNA
<213>  Artificial

<220>
<223>  Synthetic DNA

<400>  84
ggtgcccacc cagctactgc tcgcg                                             25

<210>  85
<211>  43
<212>  DNA
<213>  Artificial

<220>
<223>  Synthetic DNA

<400>  85
taatacgact cactataggt cacatccggg cgggttggtg agt                         43

<210>  86
<211>  43
<212>  DNA
<213>  Artificial

<220>
<223>  Synthetic DNA

<400>  86
tccaactatg tatacctggg tgcccaccca gctactgctc gcg                         43

<210>  87
<211>  33
<212>  DNA
<213>  Artificial

<220>
<223>  Synthetic DNA

<400>  87
ctagcctcga gaatttcaca tccgggcggg ttg                                    33

<210>  88
<211>  33
<212>  DNA
<213>  Artificial

<220>
<223>  Synthetic DNA

<400>  88
ccatggtggc gaattgtttt cccagtcacg acg                                    33


<210>  89
<211>  39
<212>  DNA
<213>  Artificial

<220>
<223>  Synthetic DNA

<400>  89
gatccccgct actgctcgcg ccggaactca ccattttta                              39


<210>  90
<211>  39
<212>  DNA
<213>  Artificial

<220>
<223>  Synthetic DNA

<400>  90
agcttaaaaa tggtgagttc cggcgcgagc agtagcggg                              39

## Description

### Technical Field

The present invention relates to a method for suppressing protein translation reaction by inducing a guanine-quadruplex structure to be formed on a target mRNA via sequence-selective binding to the target mRNA sequence so as to inhibit peptide synthesis reaction by ribosomes.

### Background Art

Various gene expression suppression techniques such as microRNA and siRNA are known^{1,2} (Non Patent Literature 1 and 2). However, in any of such technologies, since the gene expression-suppressing function is exerted by a simple mechanism of binding to mRNA, unexpected effects (off-target effects) are unavoidable³ (Non Patent Literature 3). In addition, when using a modified nucleic acid for the purpose of improving the intracellular stability of an introduced nucleic acid, the microRNA effect and siRNA activity are significantly reduced, and there is a problem that the expected protein expression-suppressing effect cannot be obtained⁴ (Non Patent Literature 4).

### Citation List

### Non Patent Literature

Non Patent Literature 1: Fire, A.; Xu, S.; Montgomery, M. K.; Kostas, S. A.; Driver, S. E.; Mello, C. C. Nature 1998, 391, 806.
Non Patent Literature 2: Ambros, V. Nature 2004, 431, 350.
Non Patent Literature 3: Jackson, A. L.; Linsley, P. S. Nature reviews. Drug discovery 2010, 9, 57.
Non Patent Literature 4: Jackson, A. L.; Burchard, J.; Leake, D.; Reynolds, A.; Schelter, J.; Guo, J.; Johnson, J. M.; Lim, L.; Karpilow, J.; Nichols, K.; Marshall, W.; Khvorova, A.; Linsley, P. S. Rna 2006, 12, 1197.

### Summary of Invention

### Technical Problem

Modified nucleic acids that can be stable *in vivo* are available to develop nucleic acid drugs with a view to drug discovery, and it is required to adopt protein translation suppression technology that does not easily cause off-target effects.

As a result of assiduous studies made for the purpose of solving the above-mentioned problems, the present inventors succeeded in forming a guanine-quadruplex sequence-selectively with the use of an oligomer called "Staple nucleic acid," thereby suppressing protein synthesis. This has led to the completion of the present invention.

### Solution to Problem

Specifically, the present invention is as follows.
(1) A method for suppressing protein expression, comprising using an oligomer for a target RNA comprising first, second, third, and fourth guanine repeat sequences (referred to as "first G sequence," "second G sequence," "third G sequence," and "fourth G sequence," respectively), the oligomer hybridizing with regions proximal to the guanine repeat sequences, so as to bring the guanine repeat sequences closer together via folding of the oligomer, thereby forming a guanine-quadruplex structure, wherein a nucleic acid of a part of a region on one end side (one end region) and a nucleic acid of a part of a region on the other end side (other end side region) of the oligomer hybridize with a region that is between the first G sequence and the second G sequence and proximal to both the first G sequence and the second G sequence, a region that is between the second G sequence and the third G sequence and proximal to both the second G sequence and the third G sequence, or a region that is between the third G sequence and the fourth G sequence and proximal to both the third G sequence and the fourth G sequence.
(2) The method according to (1), wherein the oligomer is designed such that its folding direction faces the guanine quadruplex structure side for hybridization.
(3) The method according to (1) or (2), wherein the target RNA is mRNA, TPM3, or MYD88.
(4) The method according to any one of (1) to (3), wherein the oligomer is DNA or RNA.
(5) The method according to any one of (1) to (4), wherein the oligomer is a dimer.
(6) The method according to any one of (1) to (5), wherein the protein expression is reverse transcription reaction or protein translation reaction.
(7) An oligomer for bringing first, second, third, and fourth guanine repeat sequences (referred to as "first G sequence," "second G sequence," "third G sequence," and "fourth G sequence," respectively) included in a target RNA closer together so as to form a guanine-quadruplex structure, wherein a nucleic acid of a part of a region on one end side (one end region) and a nucleic acid of a part of a region on the other end side (other end side region) of the oligomer hybridize with a region that is between the first G sequence and the second G sequence and proximal to both the first G sequence and the second G sequence, a region that is between the second G sequence and the third G sequence and proximal to both the second G sequence and the third G sequence, or a region that is between the third G sequence and the fourth G sequence and proximal to both the third G sequence and the fourth G sequence such that the guanine repeat sequences are brought closer together via folding of the oligomer.
(8) The oligomer according to (7), which is designed such that its folding direction faces the guanine quadruplex structure side for hybridization.
(9) The oligomer according to (7) or (8), which is DNA or RNA.
(10) The oligomer according to any one of (7) to (9), which is a dimer.
(11) A method for producing the oligomer according to any one of (7) to (10), comprising designing and synthesizing the oligomer such that a nucleic acid of a part of a region on one end side (one end region) and a nucleic acid of a part of a region on the other end side (other end side region) of the oligomer hybridize with a region that is between the first G sequence and the second G sequence and proximal to both the first G sequence and the second G sequence, a region that is between the second G sequence and the third G sequence and proximal to both the second G sequence and the third G sequence, or a region that is between the third G sequence and the fourth G sequence and proximal to both the third G sequence and the fourth G sequence.
(12) A kit for suppressing protein expression, comprising the oligomer according to any one of (7) to (10) or an oligomer produced by the method according to (11).

### Effects of Invention

By the method developed according to the present invention for designing an oligomer that sequence-selectively binds to (hybridizes with) a target mRNA, a stable guanine-quadruplex structure is induced in the target mRNA using the designed oligomer, thereby making it possible to inhibit peptide synthesis reaction by ribosomes.

### Brief Description of Drawings

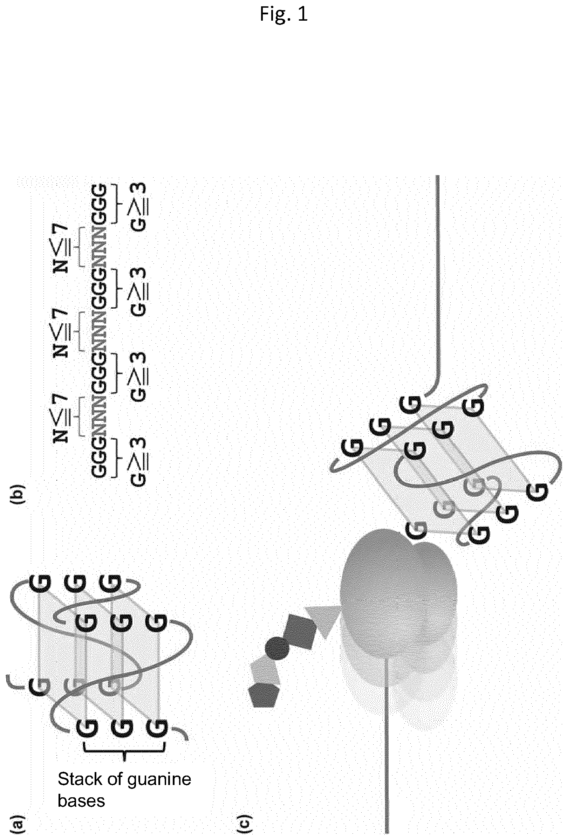

Figure 1 shows schematic view of guanine-quadruplex (G-quadruplex). (a) G-quadruplex structure. A G-quadruplex structure is formed by bringing four guanine repeat regions close to each other. (b) Rules for forming G-quadruplex. It is said that according to the higher-order structure prediction based on the thermal stability of nucleic acid, in a case where guanine repeats 3 bases or more and the number of bases joining guanine repeat sequences is 7 bases or less, a G-quadruplex is constructed. (c) It is known that construction of a G-quadruplex in mRNA inhibits the peptide elongation reaction of ribosome, resulting in suppression of the protein synthesis reaction.

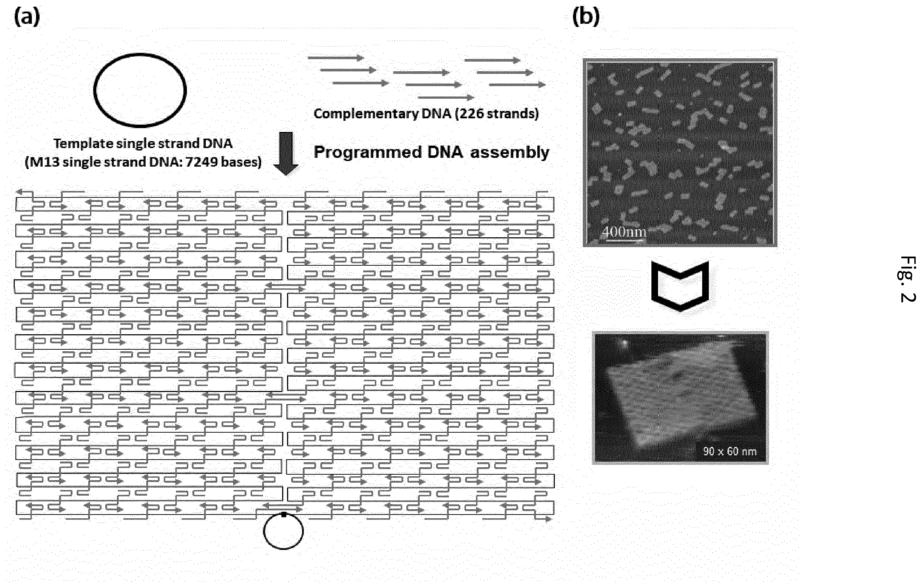

Figure 2 shows schematic view of the DNA origami method.
   (a) Any DNA nanostructure can be made by mixing 226 short DNA strands with single-strand circular DNA and annealing. (b) Results of observing the prepared nanostructure with a high-speed atomic force microscope (high-speed AFM)

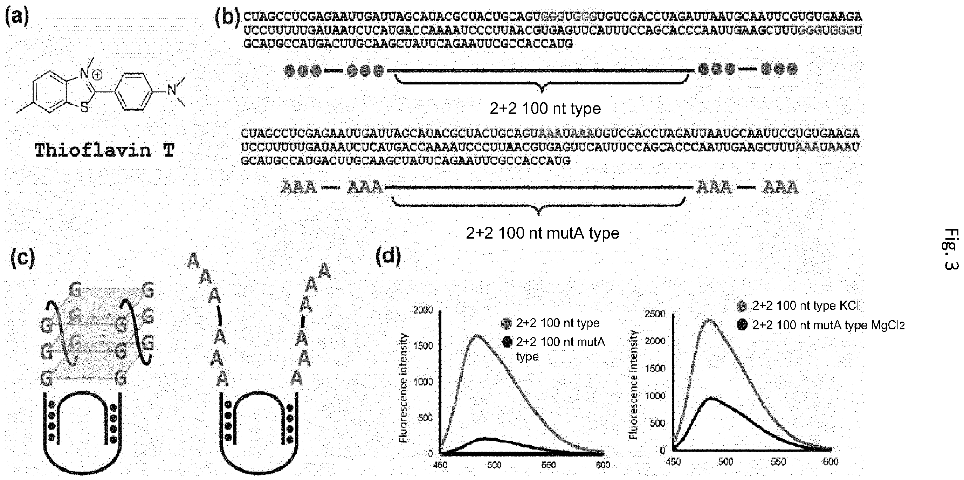

Figure 3 shows examination results of confirming the formation of G-quadruplex using Thioflavin T. (a) Chemical structural formula of Thioflavin T. (b) 2+2 100nt-type and 2+2 100nt mutA-type template RNA sequences. Red letter: Guanine repeat sequences brought close to each other, and adenine repeat sequences. (c) Structural image after introduction of Staple nucleic acid A G-quadruplex is constructed by bringing guanine repeat sequences close to each other. (d) Results of measuring fluorescence signals.

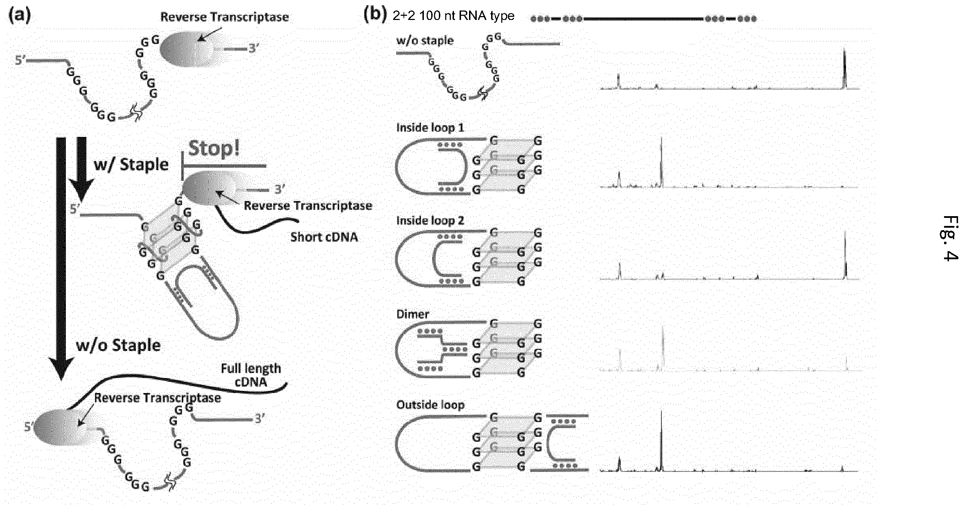

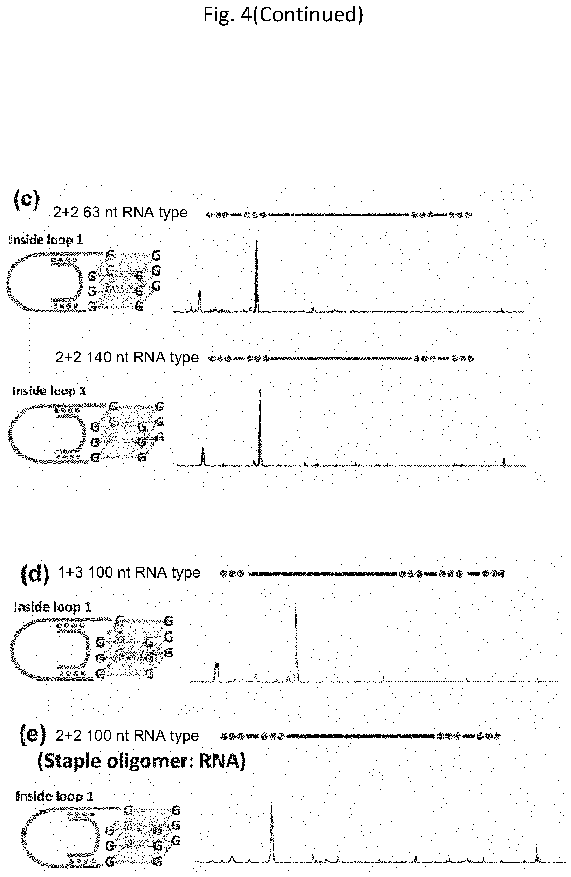

Figure 4 shows schematic view of stop assay evaluation. (a) Principles of stop assay RNA G-quadruplex arrests reverse transcriptase elongation reaction. By decoding the base sequence of cDNA formed up to the position where the reverse transcriptase stopped, the position where an RNA G-quadruplex is constructed can be confirmed. (b) Results of stop assay using 2+2 100nt RNA. Except for inside loop 2, a remarkable peak indicating the presence of RNA G-quadruplex could be confirmed. (c) Results of stop assay when the length of the loop base joining guanine bases for stacking is changed. (d) Results of evaluating 1+3 100nt RNA type by stop assay (d) Results of changing the Staple nucleic acid to be introduced from DNA to RNA.

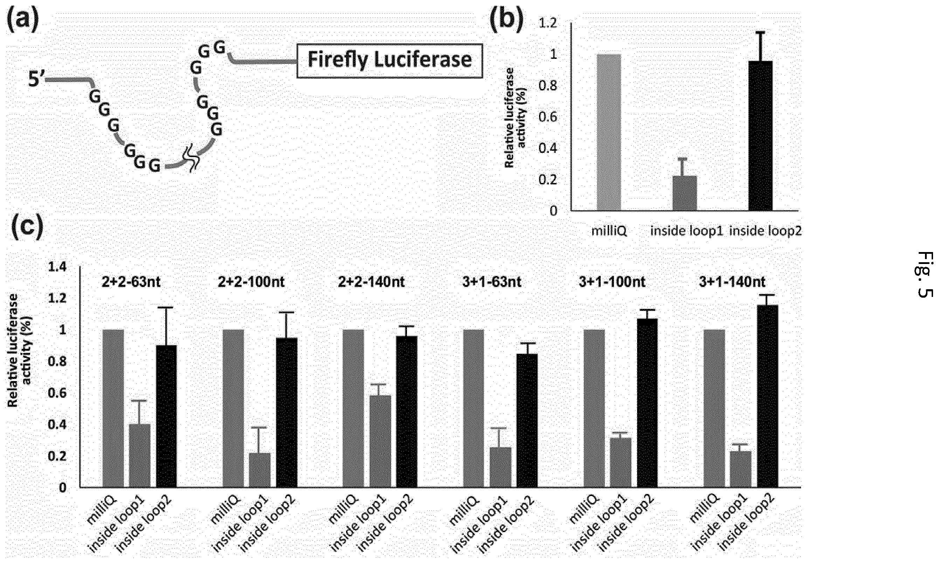

Figure 5 shows schematic view of *in vitro* translation assay. (a) Results of arranging various patterns of evaluation targets such as 2+2 100nt RNA in the 5' UTR region upstream of firefly luciferase to evaluate the effect of protein expression suppression by Staple nucleic acid. (b) Results of *in vitro* translation assay for 2+2 100nt RNA (c) Results of *in vitro* translation assay for all patterns of mRNA.

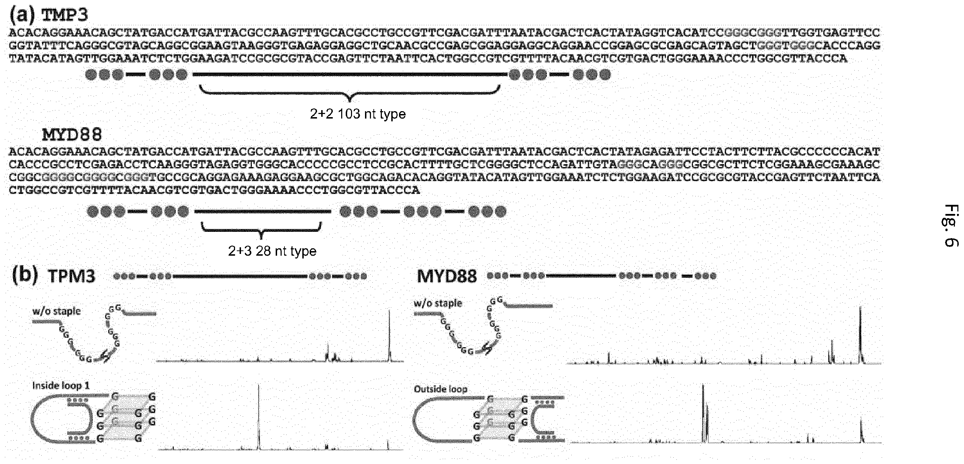

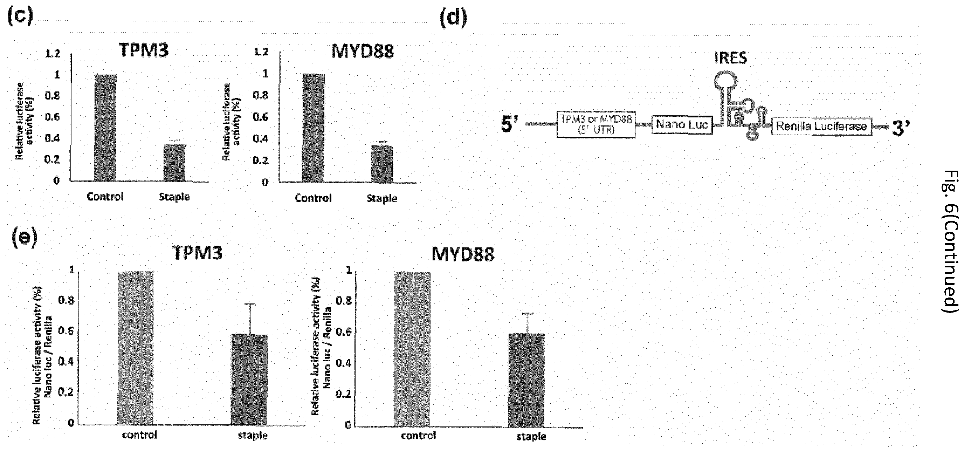

Figure 6 shows the results of evaluation experiment of Staple nucleic acid targeting natural sequences. (a) Sequences of TPM3 and MYD88 used for evaluation. (b) Results of stop assay for TPM3 and MYD88. It is understood that the RNA G-quadruplex is constructed by the presence of Staple nucleic acid. (c) Results of *in vitro* translation assay targeting TPM3 and MYD88. In both cases, it can be seen that the protein expression level is reduced due to the presence of Staple nucleic acid. (d) Design of an evaluation target for functional evaluation of Staple nucleic acid in cells. The presence of IRES allows ribosomes to invade from two locations, the 5'end and the IRES, to synthesize proteins. (e) Results of intracellular protein translation assay.

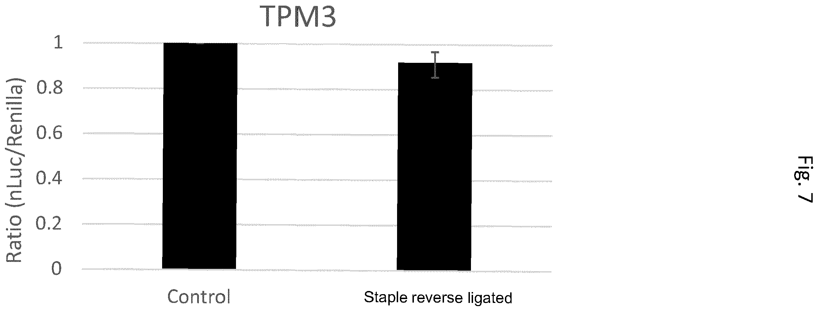

Figure 7 shows the results of the evaluation experiment when the Staple nucleic acid was reversely ligated.

### Description of Embodiments

The present invention relates to a method for suppressing protein expression by using an oligomer for RNA having four (first, second, third, and fourth) guanine repeat sequences, the oligomer hybridizing with regions proximal to the guanine repeat sequences, so as to bring the guanine repeat sequences closer together, thereby forming a guanine-quadruplex structure.

The stable guanine quadruplex structure (also referred to as "G-quadruplex structure") present on mRNA is known to inhibit the peptide elongation reaction of ribosomes. By allowing a G-quadruplex structure to be artificially induced on an arbitrary mRNA, it becomes possible to suppress the expression level of a disease-related protein. According to the present invention, there is provided a method for designing a novel nucleic acid medicine that suppresses protein translation in a target mRNA-selective manner, comprising sequence-selectively capturing and folding a target mRNA to induce a stable G-quadruplex structure on the target mRNA by applying the DNA origami technology.

In the oligomer used in the present invention, a nucleic acid of a part of a region on one end side (one end region) and a nucleic acid of a part of a region on the other end side (other end side region) each hybridize with a region that is proximal to both a first guanine repeat sequence (referred to as "first G sequence") and a second guanine repeat sequence (referred to as "second G sequence"), a region that is proximal to both the second G sequence and a third guanine repeat sequence (referred to as "third G sequence"), and a region that is proximal to both the third G sequence and a fourth guanine repeat sequence (referred to as "fourth G sequence") of RNA. This oligomer is also referred to as "Staple nucleic acid" in the present invention.

In order for the method of the present invention to function in cells, it is necessary to satisfy both the nucleic acid sequence recognition ability and the G-quadruplex structure formation ability at the same time, making it possible to realize target selectivity that surpasses conventional nucleic acid drugs. The usefulness of the present invention was demonstrated as follows by verification using model sequences: (1) RNA G-quadruplex is constructed by introducing the Staple nucleic acid regardless of the length and sequence of the guanine repeating region; and (2) the induced G-quadruplex structure suppresses the reverse transcriptase elongation reaction and ribosome protein translation reaction. In addition, it was confirmed by a cell-free expression system and expression evaluation using mammalian cells that guanine clusters present in the 5' UTR of TMP3 and MYD88 mRNAs which are cancer-related genes can be folded, thereby inhibiting protein synthesis.

In the present invention, attention is paid to the RNA G-quadruplex structure, which is an RNA quadruplex structure in a mechanism for suppressing protein translation (Figure 1 (a)). The RNA G-quadruplex structure is a stable higher-order RNA structure constructed when a guanine sequence of 3 or more continuous bases is present at 4 sites with a close spacing of less than 7 bases (Figure 1 (b))⁵. When this RNA G-quadruplex structure is present in the 5' untranslated region (5' UTR) of mRNA, it suppresses the protein synthesis reaction by ribosome (Figure 1 (c))⁶.

In recent years, the present inventors have found that contiguous sequences of guanine present with a spacing of 7 bases or more construct an RNA G-quadruplex structure⁷. This fact indicates that due to the higher-order structure formed by mRNA, continuous sequences of guanine located at distant positions in the primary sequence are spatially brought close to each other to form an RNA G-quadruplex structure. Based on this finding, the present inventors have devised a method of the present invention in which continuous sequences of guanine existing at distant positions are spatially brought close to each other to induce an artificial RNA G-quadruplex structure on a target mRNA.

Incidentally, the DNA origami method, which is a technique for creating a nanostructure by allowing a single-stranded circular DNA exceeding 7,000 bases to be self-assembled with about 200 designed short-stranded DNAs (Staple oligomers) of more than 30 bases, is known (Figure 2)⁸. Staple oligomers of this technology pull sequences existing at distant positions of the single-stranded circular DNA together so as to form a compact structure. By following this technique and designing and introducing a Staple nucleic acid that brings continuous sequences of guanine on a target mRNA close to each other, it is expected to induce a G-quadruplex structure on the target mRNA, thereby making it possible to suppress the protein translation reaction. In view of this, according to the present invention, the DNA origami method, which is a method for producing a DNA nanostructure, can be applied to artificially induce an RNA G-quadruplex structure.

As described above, a G-quadruplex structure having four guanine repeat regions existing close to each other is formed (Figure 1 (a)). In the present invention, a predetermined oligomer is used to bring the four guanine repeat regions present on RNA closer together. This oligomer functions to bring the first guanine repeat sequence and the second guanine repeat sequence, the second guanine repeat sequence and the third guanine repeat sequence, or the third guanine repeat sequence and the fourth guanine repeat sequence close to each other such that the four guanine repeat sequences are brought closer together as a whole, and the oligomer is herein referred to as "Staple nucleic acid."

Examples of the target RNA used herein include mRNA, TPM3, and MYD88. TPM3 is a gene associated with muscle contraction, and MYD88 is an adapter protein that mediates Toll and interleukin (IL) 1 receptor signaling.

Guanine repeat sequences contained in the target RNA are not limited to 4 locations, and may be contained in 5 or more locations. In addition, the first, second, third, and fourth guanine repeat sequences mean four sequences arbitrarily selected from four or more guanine repeat sequences, and do not necessarily mean that guanine repeat sequences at 4 consecutive locations form a G-quadruplex structure. For example, assuming that there are guanine repeat sequences at 6 locations in one RNA, a G-quadruplex structure may be formed by the first, second, third, and fourth guanine repeat sequences in order from the 5' side, or a G-quadruplex structure may be formed by the first, second, fifth, and sixth guanine repeat sequences. In short, the locations of four guanine repeat units do not matter as long as they form a G-quadruplex structure and suppress RNA protein expression.

Here, in the present invention, suppression of protein expression includes, for example, suppression of reverse transcription reaction from mRNA, suppression of translation reaction from RNA to protein, and the like.

Figure 3 (b) shows an aspect in which the first and second guanine repeats (the first and second "•••" marks from the left in the figure), as well as the third and fourth guanine repeats (the third and fourth "•••" marks from the left in the figure), are in close proximity to each other, with the second and third guanine repeats having a length of 100 nucleotides. In Figure 3 (b), given that the first guanine repeat sequence from the left is a first G sequence, the second guanine repeat sequence from the left is a second G sequence, the third guanine repeat sequence from the left is a third G sequence, and the fourth guanine repeat sequence from the left is a fourth G sequence, since the second G sequence and the third G sequence are separated from each other, the region therebetween is folded such that the second G sequence and the third G sequence are brought close to each other.

Thus, the Staple nucleic acid is designed such that the nucleic acid in a part of the one end side (for example, the 3' side) hybridizes with a nucleic acid in a region immediately downstream of the second G sequence, and the nucleic acid in a part of the other end side (for example, the 5' side) hybridizes with a nucleic acid in a region immediately upstream of the third G sequence. In such a case, the nucleic acids in the one end side region and the other end side region hybridize such that the folding direction (convex side) faces the G-quadruplex structure side. The region where the Staple nucleic acid hybridizes with RNA can be designed as appropriate according to the type of RNA. For example, in a case where the Staple nucleic acid is designed for the RNA illustrated in Figure 3 (b), a region of 13 to 20 bases may be selected as a hybridizing region from the region of 13 to 20 bases downstream of the second G sequence, and a region of 13 to 20 bases may be selected as a hybridizing region from the region of 13 to 20 bases upstream of the third G sequence. The same applies when designing the Staple nucleic acid for other regions.

The Staple nucleic acid may be DNA or RNA. The Staple nucleic acid has a length of 26 to 40 nucleotides. The length of the region where the Staple nucleic acid hybridizes with RNA, which is the length of either the one end side region or the other end side region, may be the same or different, and is not limited. The Staple nucleic acid can be easily obtained by using a commercially available nucleic acid synthesizer.

Figure 4 is a view for illustrating an aspect in which the Staple nucleic acid designed in this way is used to bring the guanine repeat sequences close together. In Figure 4 (b), in the aspect of "Inside loop 1," a part of each end of the Staple nucleic acid hybridizes with RNA, and the folding direction of the Staple nucleic acid faces the guanine quadruplex structure side. In Figure 4, the folding direction is shown in a loop for easy understanding. The Staple nucleic acid of the present invention is turned around the boundary between one region that hybridizes with RNA and the other region as a fulcrum. In Figure 4 (b), the aspect of "Outside loop" is the sane as Inside loop 1. A part of each end of the Staple nucleic acid hybridizes with RNA, and the folding direction (the convex side (outer peripheral side) of the part displayed in a loop) faces the G-quadruplex structure side. In addition, according to the present invention, the Staple nucleic acid does not need to be a single strand, and two or more strands can bind or hybridize to form a dimer, trimmer, or the like. In Figure 4 (b), in the aspect of "Dimer," a dimer is composed of two Staple nucleic acids, and regions that do not hybridize with RNA (one or more bases) hybridize so as to form a folded region (the folded region is exemplified by multiple bases in the figure), thereby forming the dimer as a whole. In this dimer-type Staple nucleic acid, a part of the region on each side hybridizes with RNA, and the folded portion faces the G-quadruplex structure side.

The reaction between the Staple nucleic acid designed as described above and RNA, that is, the formation reaction of the G-quadruplex structure can be carried out, for example, by mixing the Staple nucleic acid in a solution containing RNA, followed by annealing. Alternatively, the Staple nucleic acid may simply be mixed in a solution containing RNA. The reaction can also be performed by introducing a short hairpin expression vector and expressing the Staple nucleic acid from inside the cell. The reaction time of RNA and the Staple nucleic acid is, for example, 1 minute (test tube experiment) to 2 days (cell experiment), and the reaction temperature is, for example, 24°C to 55°C. The formation of the G-quadruplex structure can be confirmed by, for example, the *in vitro* translation assay explained in the Examples, a fluorescence signal measurement method by reacting with thioflavin T, a stop assay method, or the like.

Since the Staple nucleic acid designed by the method of the present invention is used for protein expression suppression, it can be included in a protein expression suppression kit. Accordingly, the present invention provides a Protein expression suppression kit including the oligomer (Staple nucleic acid). In addition to the above oligomers, the kit of the present invention can include a buffer solution, a reagent for PCR, a reagent for *in vitro* translation, luciferase, an instruction manual, and the like.

### Examples

Hereinafter, the present invention will be described in more detail with reference to the Examples below. However, the scope of the present invention is not limited to these examples.

### [Example 1]

### 1. Materials

### (1) DNA oligonucleotide

DNA oligonucleotides were purchased from Eurofins Genomics K.K., Integrated DNA Technologies, and Thermo Fisher Scientific. All samples were OPC-purified or desalted and used for confirming the formation and presence/location of RNA G-quadruplex or evaluating the *in vitro* protein translation reaction inhibitory effects.

### 2. Methods

### (1) Preparation of 2+2_100nt RNA and 2+2_100nt_Mut A RNA and confirmation of RNA G-quadruplex formation using Thioflavin T

Annealing/extension were performed using the following nucleotides.
5'-TAATACGACTCACTATAGATTAGCATACGCTACTGCAGATGCGC-3'(SEQ ID NO: 1)
5'-GCTGAATAGCTTGCAAGTCATGGCATGCGCATCTGCAGTAGCGT-3'(SEQ ID NO: 2)

PCR amplification was performed using the extended nucleotide as a template and the following primers. The obtained PCR product was cloned into a pMD19 vector (Takara Bio Inc.).
Forward primer: 5'-TAATACGACTCACTA TAG-3' (SEQ ID NO: 3)
Reverse primer: 5'-TCCAACTATGTATACCTGCTGAATAGCTTGCAAGTC-3' (SEQ ID NO: 4)

PCR amplification was performed using the plasmid DNA prepared above as a template and the following primers, thereby preparing insert DNA.
Forward primer: 5'-CTATAGTGAGTCGTATTAAATCGTCGAACGGCAGG-3' (SEQ ID NO: 5)
Reverse primer: 5'-CAGGTATA CATAGTTGGAAATCTCTGGAAGATCCG-3' (SEQ ID NO: 6)

In addition, PCR amplification was performed using nucleotides having the following sequences as templates and primers, thereby preparing a linear vector. Template: 
5'-ACACAGGAAACAGCTATGACCATGATTACGCCAAGTTTGCACG
CCTGCCGTTCGACGATTTAATACGACTCACTATAGATTAGCATACGCTACTG
CAGTGGGTGGGTGTCGACCTAGATTAATGCAATTCGTACGAAGTTCATAGC
ATTTCCAGCACCCAATTGAAGCTTTGGGTGGGTGCATGCCATGACTTGCAAG
CTATTCAGCAGGTATACATAGTTGGAAATCTCTGGAAGATCCGCGCGTACC
GAGTTCTAATTCACTGGCCGTCGTTTTACAACGTCGTGACTGGGAAAACCCT
GGCGTTACCCA-3' (SEQ ID NO: 7)
Forward primer: 5'-TAATACGACTCACTATAGAA-3' (SEQ ID NO: 8)
Reverse primer: 5'-TCCAACTATGTATAC CTG-3' (SEQ ID NO: 9)

Then, the insert DNA prepared using HiFi Assembly (NEB) and the linear vector were ligated (pMD19-2+2_63nt). PCR amplification was performed using a pUC19 vector (Takara Bio Inc.) as a template and the following primers, thereby preparing insert DNA.

Forward primer: 5'-CCTAGATTAATGCAATTC
GTGTGAAGATCCTTTTTGATAATCTCATGAC-3' (SEQ ID NO: 10)

Reverse primer: 5'-TCAATTGGGTGCT
GGAAATGAACTCACGTTAAGGGATTTTGGTCATGAG-3' (SEQ ID NO: 11)

In addition, PCR amplification was performed using pMD19-2+2_63nt as a template and the following primers, thereby preparing a linear vector.
Forward primer: 5'-CATTTCCA GCACCCAATTGAAGCTT-3' (SEQ ID NO: 12)
Reverse primer: 5'-ACGAATTGCATTAATCTAG GTCGAC-3' (SEQ ID NO: 13)

Then, the insert DNA prepared using HiFi Assembly (NEB) and the linear vector were ligated (pMD19-2+2_100nt).

2+2_100nt was amplified by PCR using the prepared pMD19-2+2_100nt as a template and the following primers, and the amplified product was cloned into the EcoR I site of a pIRES vector (Takara Bio Inc.).
Forward primer: 5'- CTAGCCTCGAGAATTGATTAGCAT ACGCTACTGC-3' (SEQ ID NO: 14)
Reverse primer: 5'- CCATGGTGGCGAATTCTGAATAGCTTGCAAGTC AT-3' (SEQ ID NO: 15)

For 2+2_100nt_Mut A, a mutant from GGG to AAA, annealing/extension were performed using, as a template DNA, a forward primer, and a reverse primer, the following sequences, respectively, and the obtained products were ligated by PCR (2+2_100nt_MutA).
(i) MutA-1
   Template: 5'-AGGGATTAGCATACGCTACTGCAGTAAAT AAATGTCGACCTAG-3' (SEQ ID NO: 16) 
Forward primer: 5'-CGGTACCCGGGGATCTAATACGACTCACTATAGGGAT
TAGC-3' (SEQ ID NO: 17)
   Reverse primer: 5'-CACGAATTGCATTAATCTAGGTCGAC-3' (SEQ ID NO: 18)
(ii) MutA-2
   Template: pMD19-2+2_100nt
   Forward primer: 5'-GTCGACCTAGATTAATGCAATTCGTG-3' (SEQ ID NO: 19)
   Reverse primer: 5'-AAGCTTCAATTGGGTGCTGGAA ATG-3' (SEQ ID NO: 20)
(iii) MutA-3
   Template: 5'-ATTGAAGCTTTAAATAAATGCATGCCATGACTTGCAAG-3' (SEQ ID NO: 21)
   Forward primer: 5'-CATTTCCAGCACCCAATTGAAGCTT-3' (SEQ ID NO: 22)
   Reverse primer: 5'-CGACTCTAGAGGATCCTGAATAGCTT GCAAGTCAT-3' (SEQ ID NO: 23)

The resulting product was cloned into the BamH I site of a pUC19 vector (Takara Bio Inc.) (pUC19-2+2_100nt_Mut A). Each PCR product was excised and purified after 1% agarose gel electrophoresis. PCR amplification was performed using the prepared pIRES-2+2_100nt and pUC19-2+2_100nt_Mut A as a template and any one of the following primers, thereby prepared linear dsDNAs for RNA transcription.
Forward primer: 5'-TAATACGACTCACTATAGG GCTAGCCTCGAG-3' (SEQ ID NO: 24)
Reverse primer: 5'-CCATGGTGGCGAATTCTGAATAGCTTGCAAGTCAT-3' (SEQ ID NO: 25)
   or
Forward primer: 5'-TAATACGACTCACTATAGGG-3' (SEQ ID NO: 26)
Reverse primer: 5'-CGACTCTAGAGGATCCTGAATAG CTTGCAAGTCAT-3' (SEQ ID NO: 27)

Further, the prepared dsDNAs were used as a template so as to transcribe RNAs from dsDNAs with a RiboMAX (trademark) Large Scale RNA Production System-T7 (Promega). RNAs were purified using a NAP-5 column, thereby removing the remaining NTPs. A measurement solution was prepared such that it was composed of prepared RNA (400 nM), KCl (40 mM), 20 mM Tris-HCl buffer (pH 7.6), Staple (500 nM) or milliQ (control), incubated at 90°C for 2 minutes, and cooled to 20°C at a rate of 1°C per minute. Thereafter, ThT was added to yield 200 nM ThT and incubated at room temperature for 30 minutes. Excitation was performed with light at 440 nm using an FP-8500 spectrofluorometer (JASCO) so as to measure fluorescence at 487 nm. Measurement was carried out with a measurement wavelength in a range of 450 nm to 700 nm.

### (2) Stop Assay using 5' UTR sequences of artificial sequences (2+2_100nt) and natural genes (TPM3, MYD88)

PCR amplification of TPM3-5' UTR and MYD88-5' UTR was performed using human genomic DNA (Novagen) as a template and the following primers.
Forward primer: 5'-GTCACATCC GGGCGGGTTGGTGAGT-3' (SEQ ID NO: 28)
Reverse primer: 5'-GGTGCCCACCCAGCTACTGCTCGCG-3' (SEQ ID NO: 29) or
Forward primer: 5'-AGATTCCTACTTCTTACGCCCCCCA-3' (SEQ ID NO: 30)
Reverse primer: 5'-TGTCTGCCAGCGCTTCCTCTTTCTC-3' (SEQ ID NO: 31)

PCR amplification was further performed using the obtained PCR product as a template and the following primers, and the thus obtained PCR product was cloned into a pMD19 vector (Takara Bio Inc.) (pMD19-TPM3, pMD19-MYD88).
Forward primer: 5'-TAATACGACTCACTATAGGTCACATCCGG GCGGGTTGGTGAGT-3' (SEQ ID NO: 32)
Reverse primer: 5'-TCCAACTATGTATACCTGGGTGCCCACCCAGCTACT GCTCGCG-3' (SEQ ID NO: 33)
   or 
Forward primer: 5'-TAATACGACTCACTATAGAGATTCCTACTTCTTACGCCCCC
CA-3' (SEQ ID NO: 34)
Reverse primer: 5'-TCCAACTATGTATACCTGTGTCTGCCAGCGCTTCCTCTTTCTC-3' (SEQ ID NO: 35)

PCR amplification was performed using pMD19-5' UTR (2+2_100nt, TPM3, MYD88) as a template and the following primers, thereby preparing linear dsDNAs for RNA transcription.
Forward primer: 5'-ACACAGGAAACAGCTATGACCATGATTACGC CAAG-3' (SEQ ID NO: 36)
Reverse primer: 5'-TGGGTAACGCCAGGGTTTTCCCAGTCACGACGTTG-3' (SEQ ID NO: 37)

Further, the prepared dsDNAs were used as a template so as to transcribe RNAs from dsDNAs with a HiScribe T7 High Yield RNA Synthesis Kit (NEB). The transcribed RNAs were purified using an After Tri-Reagent RNA Clean-Up Kit (Chiyoda Science Co., ltd.).

A measurement solution was prepared such that it contained RNA (0.3 µM), a 5'-FAM-labeled 3'-DNA primer (0.1 µM), and Staple (1 µM), incubated at 80°C for 3 minutes, and cooled to 30°C. ReverTra Ace reverse transcriptase (TOYOBO), MgCl₂, and dNTPs were added to the solution, and a reverse transcription reaction was carried out for 30 minutes. The obtained reverse transcript was examined by fragment analysis using a ABI3500 Capillary DNA Sequencer (Life Technologies Japan Ltd.).

### (3) In vitro translation using artificial sequence (2+2_100nt) and 5' UTR sequences of natural genes (TPM3, MYD88)

Firefly luciferase (F. L.) was amplified by PCR using a psiCHECK-2 vector (Takara Bio Inc.) as a template and the following primers. The obtained PCR product was cloned into the EcoR I site of a pIRES vector (Takara Bio Inc.) (pIRES-F. L.).
Forward primer: 5'-CTAGCCTCGAGAATT CGCCACCATGGCCGATGCTAAGAAC-3' (SEQ ID NO: 38)
Reverse primer: 5'- CTCGACGCGTGAATTTT ACACGGCGATCTTGCC-3' (SEQ ID NO: 39)

The forward primer was designed such that the EcoR I site was restored upstream of F. L. after cloning. For 5' UTR (2+2_100nt, TPM3, MYD88), PCR amplification was performed using pMD19-5'UTR (2+2_100nt, TPM3, MYD88) as a template and the following primers. The obtained PCR products were each cloned into the EcoR I site of a pIRES-F. L. vector (pIRES-5'UTR-F. L. (5'UTR: 2+2_100nt, TPM3, MYD88)).
Forward primer: 5'-CTAGCCTCGAGAATTGAT TAGCATACGCTACTGC-3' (SEQ ID NO: 40)
Reverse primer: 5'-CCATGGTGGCGAATTCTGAATAGCTTGCAAGTC AT-3' (SEQ ID NO: 41)
   or
Forward primer:
   5'-CTAGCCTCGAGAATTTCACATCCGGGCGGGTTG-3' (SEQ ID NO: 42) or
   5'-CTAGCCT CGAGAATTAGATTCCTACTTCTTACGCC-3' (SEQ ID NO: 43)
Reverse primer: 5'-CCATGGTGGCGAATTGTTTTCCC AGTCACGACG-3' (SEQ ID NO: 44)

Each PCR product was excised and purified after 1% agarose gel electrophoresis. PCR amplification was performed using pIRES-5'UTR-F. L. as a template and the following primers, thereby preparing linear dsDNAs for RNA transcription.
Forward primer:
   5'-TAATACGACTCACTATAGGGCTAGCCT CGAG-3' (SEQ ID NO: 45) 5'-TAATACGACTCACTATAGGGTCACATCCGGG-3' (SEQ ID NO: 46) or
   5'-TAATACGACTCACTATAGGGAGATTCCTAC-3' (SEQ ID NO: 47)
Reverse primer: 5'-CTCGACGCGTGAAT TTTACACGGCGATCTTGCC-3' (SEQ ID NO: 48)

Further, the prepared dsDNAs were used as a template so as to transcribe RNAs from dsDNAs with a RiboMAX (trademark) Large Scale RNA Production System-T7 (Promega). RNAs were purified using a NAP-5 column (GE Healthcare), thereby removing the remaining NTPs. The obtained 5' UTR-F. L. (5' UTR: 2+2_100nt or TPM3 or MYD88) was used as mRNA (1µg) in 12.5 µL of a cell-free protein synthesis reaction solution (RTS 100 Wheat Germ Kit, biotechrabbit) containing Staple (100 nM) or milliQ (control). This reaction solution was incubated at 24°C for 1 hour, and then evaluated for luciferase activity using a Luciferase Assay kit (Promega) and a POWERSCAN·H1 microplate reader (BioTek).

### (4) In cell translation using 5' UTR sequences of natural genes (TPM3, MYD88).

NanoLuc luciferase (nLuc) and renilla luciferase (renilla) were amplified by PCR using an nLuc vector (Takara Bio Inc.) or a psiCHECK-2 vector (Takara Bio Inc.) as a template and the following primers. The obtained PCR products were cloned into the EcoR I and Not I sites of a pIRES vector (Takara Bio Inc.), respectively (pIRES-nLuc-renilla).
Forward primer: 5'-CTAGCCTCGAGAATTCGCCACCATGGTCTTCACACTCGAAG-3' (SEQ ID NO: 49)
Reverse primer: 5'-CTAGCCTCGAGAATTCGCCACCATGGTCTTCACACTCGAAG-3' (SEQ ID NO: 50)
   or
Forward primer: 5'-TCGACCCGGGCGGCCATGGCTTCCAAGGTGTACG-3' (SEQ ID NO: 51)
Reverse primer: 5'-TAAAGGGAAGCGGCCTTACTGCTCGTTCTTCAGC-3' (SEQ ID NO: 52)

The forward primer used for PCR of nLuc was designed such that the EcoR I site was restored upstream of nLuc after cloning. Each PCR product was excised and purified after 1% agarose gel electrophoresis. Further, PCR amplification was performed using pMD19-TPM3 or pMD19-MYD88 as a template and the following primers, and the obtained PCR product was cloned into the EcoR I site of pIRES-nLuc-renilla (pIRES-5'UTR-nLuc-renilla (5'UTR: TPM3, MYD88)).
Forward primer: 5'-CTAGCCTCGAGAATTTCACATCCGGGCGGGTTG-3' (SEQ ID
NO: 53) or 5'-CTAGCC TCGAGAATTAGATTCCTACTTCTTACGCC-3' (SEQ ID NO: 54)
Reverse primer: 5'-CCATGGTGGCGAATT GTTTTCCCAGTCACGACG-3' (SEQ ID NO: 55)

In addition, annealing/extension were performed using any of the following primer combinations, thereby preparing Staple (TPM3_30nt, 26nt and MYD88_30nt, 26nt).
(i) Primer STM1 
Forward primer: 5'-GATCCCCTACCGGAACTCACCA
GCTACTGCTCGCGCTTTTTTA-3' (SEQ ID NO: 56)
 
Reverse primer: 5'-AGCTTAAAAAGCGCGAGCAGTAGCTGGT
GAGTTCCGGTAGGG-3' (SEQ ID NO: 57)
(ii) Primer STM2
   Forward primer: 5'-GATCCCCCCGGAACTCACCAGCTACTGCTCGCG TTTTTA-3' (SEQ ID NO: 58)
   Reverse primer: 5'-AGCTTAAAAACGCGAGCAGTAGCTGGTGAGTTCCGGGGG-3' (SEQ ID NO: 59)
(iii) Primer STM3
   Forward primer: 5'-GATCCCCTCTATCTCCTGCGGCACAATCTGGAGCCCCTTTTTA-3' (SEQ ID NO: 60)
   Reverse primer: 5'-AGCT TAAAAAGGGGCTCCAGATTGTGCCGCAGGAGATAGAGGG-3' (SEQ ID NO: 61)
(iv) Primer STM4
   Forward primer: 5'-GATCCCCTATCTCCTGCGGCACAATCTGGAGCCTTTTTA-3' (SEQ ID NO: 62)
   Reverse primer: 5'-AGCTTAAAAAGG CTCCAGATTGTGCCGCAGGAGATAGGG-3' (SEQ ID NO: 63)

The base sequences of the prepared Staple nucleic acids are described below.
Staple (40nt) 2+2_100nt used in the ThT experiment:
   insideloop TTGCATTAATCTAGGTCGACAAGCTTCAATTGGGTGCTGG (SEQ ID NO: 64)
Staple 2+2_100nt used in Stop assay:
   insideloop 40nt TTGCATTAATCTAGGTCGACAAGCTTCAATTGGGTGCTGG (SEQ ID NO: 65)
2+2_100nt:
   insideloop_reverse-ligated 40nt AAGCTTCAATTGGGTGCTGGTTGCATTAATCTAGGTCGAC (SEQ ID NO: 66)
TPM3: DNA Staple insideloop 40nt GAAATACCGGAACTCACCAAAGCTACTGCTCGCGCTCCGG (SEQ ID NO: 67)
MYD88: DNA Staple outsideloop 40nt TTCCTCTTTCTCCTGCGGCATACAATCTGGAGCCCCGAGC (SEQ ID NO: 68)
2+2_100nt: RNA Staple insideloop 30nt UUAAUCUAGGUCGACAAGCUUCAAUUGGGU (SEQ ID NO: 69)
2+2_100nt: RNA Staple insideloop 26nt AAUCUAGGUCGACAAGCUUCAAUUGG (SEQ ID NO: 70)

Staple used for *in vitro* translation (40nt)
2+2_100nt: insideloop TTGCATTAATCTAGGTCGACAAGCTTCAATTGGGTGCTGG (SEQ ID NO: 71)
2+2_100nt: insideloop_reverse-ligated AAGCTTCAATTGGGTGCTGGTTGCATTAATCTAGGTCGAC (SEQ ID NO: 72)
TPM3: insideloop TGAAATACCGGAACTCACCAGCTACTGCTCGCGCTCCGGT (SEQ ID NO: 73)
MYD88: outsideloop CTTCCTCTTTCTCCTGCGGCACAATCTGGAGCCCCGAGCA (SEQ ID NO: 74)

RNA Staple TPM3 used for in cell translation:
RNA Staple insideloop 30nt UACCGGAACUCACCAGCUACUGCUCGCGCU (SEQ ID NO: 75)
TPM3: RNA Staple insideloop 26nt CCGGAACUCACCAGCUACUGCUCGCG (SEQ ID NO: 76)
MUD88: RNA Staple UAU outsideloop 30nt UCUAUCUCCUGCGGCACAAUCUGGAGCCCC (SEQ ID NO: 77)
MYD88: RNA Staple UAU outsideloop 26nt UAUCUCCUGCGGCACAAUCUGGAGCC (SEQ ID NO: 78)

Each of the above was cloned between the Bgl II-Hind III sites of pSuper neo. A (oligoengine) (pSuper neo. A-Staple (Staple: TPM3_30nt, 26nt and MYD88_30nt, 26nt)). HEK293T cells were cultured using medium A (Dulbecco's modified Eagle medium containing 100 units/ml penicillin, 100 µg/ml streptomycin, and 10% (v/v) fetal bovine serum) in an incubator at 37°C and a CO₂ concentration of 5%. HEK293T cells were seeded on a 96-well plate at 2 × 10⁴ cells per well.

At 24 hours later, pSuper neo. A-Staple (Staple: TPM3_30nt, 26nt, and MYD88_30nt, 26nt) or milliQ (control) was introduced into the HEK293T cells using a transfection reagent (FuGENE, Promega), and then the cells were incubated. At 48 hour later, the HEK293T cells were transfected with pIRES-5'UTR-nLuc-renilla (5'UTR: TPM3 or MYD88) and incubated in the same manner.
At 6 hours later, the cells were washed with PBS, and the cell lysate was collected using Passive Lysis Buffer. This cell lysate was centrifuged at 4°C and 3000 rpm for 1 minute. Thereafter, luciferase activity was evaluated using the supernatant and a Luciferase Assay kit (Promega) with a POWERSCAN˙H1 microplate reader (BioTek).

### Results:

Construction of a G-quadruplex structure by the Staple nucleic acid was evaluated using G-quadruplex structure using Thioflavin T, which is known to emit a fluorescent signal when bound to a G-quadruplex structure, as an indicator (Figure 3 (a))⁹. In this experiment, a model DNA sequence (2+2 100nt type), in which two guanine repeat sequences close to both ends of a 100-base loop sequence exist, was designed, and used. In addition, DNA in which guanine in the guanine repeat sequence was mutated to adenine (2+2 100nt mutA type) was used as a control (Figures 3 (b) and 3 (c)).

The Staple nucleic acid was added to the obtained DNA sequences. Accordingly, the 2+2 100nt type DNA had an increased fluorescence signal of Thioflavin T only in the presence of the Staple nucleic acid. Meanwhile, the 2+2 100nt mutA type DNA was not confirmed to have a significant fluorescence signal with or without the Staple nucleic acid. The results indicate that the Staple nucleic acid induced a G-quadruplex structure on the 2+2 100nt type DNA.

A G-quadruplex structure is constructed in a potassium ion-dependent manner¹⁰. To confirm that a structure composed of the 2+2 100nt type model sequence and the Staple nucleic acid is a G-quadruplex structure, the fluorescence signal intensity of Thioflavin T in Tris buffer containing KCl or MgCl₂ was compared for examination.

As a result, a strong fluorescent signal was observed only in Tris buffer containing KCl (Figure 3 (d)). These results indicate that the 2+2 100nt type model DNA was folded by the Staple nucleic acid, resulting in the formation of a G-quadruplex structure.

Next, ability of the Staple nucleic acid to form an RNA G-quadruplex structure was evaluated by cDNA synthesis with a reverse transcriptase (stop assay). Full-length cDNA can be obtained from the designed template RNA by reverse transcription reaction in the absence of the Staple nucleic acid. However, when the Staple nucleic acid is added, an RNA G-quadruplex structure is formed. This stops the DNA elongation reaction of the reverse transcriptase, and non-full-length cDNA is synthesized. By analyzing the obtained cDNA sample by sequencing, it is possible to confirm that the reverse transcription reaction is stopped at the RNA G-quadruplex structure forming part (Figure 4 (a))¹¹.

In this Example, a model RNA sequence (2+2 100nt type RNA), in which two guanine repeat sequences close to both ends of a 100-base loop sequence exist, and four different short DNAs (inside loop 1, outside loop, dimer, inside loop 2) were designed and used. When three different DNAs, inside loop 1, outside loop, and dimer, were added, peaks indicating the construction of RNA G-quadruplex structure were observed Meanwhile, inside loop 2, which was expected not to function as a Staple nucleic acid, did not construct an RNA G-quadruplex structure, resulting in only full-length cDNA (Figure 4(b)).

The results mean that even in a case where RNA is a template, the DNA origami technology can fold RNA as designed and induce an RNA G-quadruplex structure. In addition, two types of model RNA sequences (2+2 63nt RNA type, 2+2 140nt RNA type) with loop lengths of 63nt and 140nt were designed, and the same study using inside loop 1 was conducted. Accordingly, a peak showing RNA G-quadruplex construction was confirmed for every RNA (Figure 4 (c)).

A peak indicating the construction of the RNA G-quadruplex structure was also observed by the introduction of the Staple nucleic acid in the case of a model RNA sequence having a different pattern of guanine repeat sequences with one guanine repeat sequence at one end and three guanine repeat sequences at the other end (1+3 100nt RNA type) (Figure 4 (d)). The results indicate that various RNA species can be folded with the Staple nucleic acid so as to form an RNA G-quadruplex structure, and the method of the present invention can be used for any mRNA target. Since the Staple nucleic acid shows similar effects when using RNA as well as DNA (Figure 4 (e)), it is also possible to use an artificially modified nucleic acid having ability to form complementary strands.

It is known that the RNA G-quadruplex structure suppresses not only reverse transcription reaction but also protein translation reaction⁶. When it becomes possible to construct an artificial RNA G-quadruplex on a target mRNA using a Staple nucleic acid, the protein expression of the target gene can be suppressed, and thus, the possibilities of pharmaceutical application will also expand. In view of this, in this Example, an mRNA in which the firefly luciferase gene was introduced downstream of the 2+2 100nt RNA type 5'-UTR region (2+2 100nt RNA type _Firefly) was prepared, and the amount of protein synthesized in the presence or absence the staple nucleic acid was quantitatively evaluated using a cell-free protein translation system (Figure 5 (a)).

As a result, the expression level of the firefly luciferase protein present downstream was reduced in the presence of inside loop 1. Meanwhile, as the protein expression level was not reduced in the presence of inside loop 2, it is shown that the staple nucleic acid does not simply bind to the target mRNA to exert an antisense effect, but rather exerts the gene expression inhibitory effects by constructing an RNA G-quadruplex (Figure 5 (b)).

In addition, 2+2 63nt RNA type _Firefly, 2+2 140nt RNA type _Firefly, 1+3 63nt RNA type _Firefly, 1+3 100nt RNA type _Firefly, and 1+3 140nt RNA type _Firefly used in stop assay were also examined in the same manner. Accordingly, in each case, it was confirmed that in a case where inside loop 1 capable of forming an RNA G-quadruplex structure is added, the expression level of firefly luciferase is reduced (Figure 5 (c)). The results indicate generality for artificial induction of the RNA G-quadruplex structure by a Staple nucleic acid and suppression of protein translation reaction by the induced RNA G-quadruplex structure.

Next, the RNA G-quadruplex structure formation and gene expression-suppressing effect of the Staple nucleic acid were evaluated on the natural gene-sequences TPM3 and MYD88 (Figure 6 (a)). First, a Staple nucleic acid capable of inducing an RNA G-quadruplex structure in 5'-UTR of TPM3 and MYD88 was designed. For TPM3, an inside loop 1 type Staple nucleic acid was designed as the loop base for ligating guanine repeat sequences had 103 bases. For MYD88, an outside loop type Staple nucleic acid was designed because the loop base for ligating guanine repeat sequences was as short as 28 bases.

Evaluation by stop assay confirmed that an RNA G-quadruplex structure was induced in 5' UTR for either one of the designed Staple nucleic acids (Figure 6 (b)). Next, model mRNAs in which the 5'-UTRs of TPM3 and MYD88 were placed in the firefly luciferase gene were prepared so as to evaluate ability to suppress protein translation of Staple nucleic acid.

As a result, reporter assay using a cell-free protein translation system showed that protein translation from model each of the model mRNAs in which 5' UTR was placed as above was suppressed by approximately 60% in the presence of the Staple nucleic acid (Figure 6 (c)). The results strongly indicate that the Staple nucleic acid suppresses protein expression from naturally occurring target mRNAs.

Lastly, the protein translation inhibitory activity of the Staple nucleic acid in mammalian cells was evaluated. In conducting a cell experiment, the present inventors prepared genes expressed by NanoLuc and Renilla luciferase. Here, the two genes are introduced into a single mRNA, NanoLuc is translated from 5'-cap, while Renilla luciferase is translated from the internal ribosomal entry site (IRES). By using this system, the effects of transfection efficiency and mRNA stability can be standardized based on the expression level of the Renilla luciferase gene, and the translation reaction from 5'-cap can be compared quantitatively (Figure 6 (d))¹².

In this Example, a reporter gene in which the 5'-UTR regions of TPM3 and MYD88 were placed upstream of NanoLuc was introduced, and the effects of the Staple nucleic acid in HEK293 cells was evaluated. In the cell experiment, using an RNA Staple nucleic acid expressed by a short hairpin expression vector (pSuper), the effects of Staple nucleic acid on protein translation reaction were evaluated. As a result, it was confirmed that the amount of protein translation decreased when the Staple nucleic acid was introduced, indicating that the Staple nucleic acid also functions in cells (Figure 6 (e)).

The development of nucleic acid medicines is clearly stagnant. The cause may be that intracellular transfection is difficult and that the off-target effects cannot be avoided. While the problems related to intracellular transfection have been greatly improved by the contribution of research and development of drug delivery systems in recent years, it has been difficult to improve the off-target effects. The reason for this is that both antisense nucleic acids and siRNAs utilize complementary nucleic acids so as to bind to a target mRNA. When a modified nucleic acid that improves the recognition ability of complementary nucleic acids is introduced, siRNA immediately reduces the ability to form an RISC complex, and its effects are greatly reduced. There is a dilemma that a new problem arises when trying to solve one problem in this way.

Against this background, the present inventors have proposed a new protein translation reaction suppression system that has never existed before. In this system, even if a Staple nucleic acid binds to an unintended position, there is no guanine repeat sequences to be brought closer together, and the protein inhibitory effect is not exhibited. It is also clarified that a Staple nucleic acid to be introduced is not limited to DNA, and thus the same effects can be obtained also with RNA. This fact shows the diversity of Staple nucleic acids.

Usually, as natural nucleic acid sequences are degraded when introduced into cells, it is essential to use nucleic acids modified by chemical synthesis in order to aim for nucleic acid medicines. According to the method of the present invention, there is no need to form protein complexes like siRNA. Therefore, it is only necessary to concentrate on improving intracellular stability, and in principle, it is unlikely that modified nucleic acids will reduce the protein expression-suppressing effect. The method of the present invention, by which various modifications are possible, is extremely useful for the development of nucleic acid medicines.

### [Example 2]

### Materials & Methods (Staple_TPM3_26nt_reverse-ligated)

### (1) DNA oligonucleotide

DNA oligonucleotides were purchased from Eurofins Genomics K.K., Integrated DNA Technologies, and Thermo Fisher Scientific. All samples were OPC-purified or desalted and used for confirming the formation and presence/location of RNA G-quadruplex or evaluating the *in vitro* protein translation reaction inhibitory effects.

### (2) Method

In cell translation using 5' UTR sequences of natural genes (TPM3, MYD88) (Staple_TPM3_26nt_reverse-ligated).

### 1. Preparation of pIRES-TPM3_5'UTR_nLuc_ renilla

NanoLuc luciferase (nLuc) and renilla luciferase (renilla) were amplified by PCR using an nLuc vector (Takara Bio Inc.) or a psiCHECK-2 vector (Takara Bio Inc.) as a template and the following set of Primer 1 or Primer 2. The obtained PCR products were cloned into the EcoR I and Not I sites of a pIRES vector (Takara Bio Inc.), respectively (pIRES-nLuc-renilla).
(i) Primer 1
   Forward primer 1: fw. primer [5'-CTAGCCTCGAGAATTCGCCACCATGGTCTTCACACTCGAAG-3'] (SEQ ID NO: 79)
   Reverse primer 1: rv. primer [5'-CTCGACGCGTGAATTTTACGCCAGAATGCGTTCG-3'] (SEQ ID NO: 80)
(ii) Primer 2
   Forward primer 2: fw. primer [5'-TCGACCCGGGCGGCCATGGCTTCCAAGGTGTACG-3'] (SEQ ID NO: 81)
   Reverse primer: rv. primer [5'-TAAAGGGAA GCGGCCTTACTGCTCGTTCTTCAGC-3'] (SEQ ID NO: 82)

The fw. primer used for PCR of nLuc was designed such that the EcoR I site was restored upstream of nLuc after cloning. Each PCR product was excised and purified after 1% agarose gel electrophoresis.

PCR amplification of TPM3-5' UTR was performed using human genomic DNA (Novagen) as a template and the following primers.
fw. primer [5'-GTCACATCC GGGCGGGTTGGTGAGT-3'] (SEQ ID NO: 83)
rv. primer [5'-GGTGCCCACCCAGCTACTGCTCGCG-3'] (SEQ ID NO: 84)

PCR amplification was further performed using the obtained PCR product as a template and the following primers, and the thus obtained PCR product was cloned into a pMD19 vector (Takara Bio Inc.) (pMD19-TPM3).
fw. primer [5'-TAATACGACTCACTATAGGTCACATCCGG GCGGGTTGGTGAGT-3'] (SEQ ID NO: 85)
rv. primer [5'-TCCAACTATGTATACCTGGGTGCCCACCCAGCTACTGCTCGCG-3'] (SEQ ID NO: 86)

Further, PCR amplification was performed using pMD19-TPM3 as a template and the following primers, and the obtained PCR product was cloned into the EcoR I site of pIRES-nLuc-renilla (pIRES-TPM3_5'UTR-nLuc-renilla).
fw. Primer: [5'-CTAGCCTCGAGAATTTCACATCCGGGCGGGTTG-3'] (SEQ ID NO: 87))
rv. primer: [5'-CCATGGTGGCGAATT GTTTTCCCAGTCACGACG-3'] (SEQ ID NO: 88)

### 2. Preparation of pSuper neo. A-Staple (Staple: TPM3_26nt_reverse-ligated)

In addition, annealing was performed using the following primers, thereby preparing Staple insert (TPM3_26nt_reverse-ligated). This was cloned between the Bgl II-Hind III sites of pSuper neo. A (oligoengine) (pSuper neo. A-Staple (Staple: TPM3_26nt_reverse-ligated)).
fw. primer [5'- GATCCCCGCTACTGCTCGCGCCGGAACTCACCAT TTTTA -3'] (SEQ ID NO: 89)
rv. primer [5'- AGCTTAAAAATGGTGAGTTCCGGCGCGAGCAGTAGCGGG -3'] (SEQ ID NO: 90)

### 3. In cell translation

HEK293T cells were cultured using medium A (Dulbecco's modified Eagle medium containing 100 units/ml penicillin, 100 µg/ml streptomycin, and 10% (v/v) fetal bovine serum) in an incubator at 37°C and a CO₂ concentration of 5%. HEK293T cells were seeded on a 96-well plate at 2 × 10⁴ cells per well. At 24 hours later, pSuper neo. A-Staple (Staple: TPM3_26nt_reverse-ligated) or milliQ (control) was introduced into the HEK293T cells using a transfection reagent (FuGENE, Promega), and then the cells were incubated.

At 48 hour later, the HEK293T cells were transfected with pIRES-TPM3_5'UTR-nLuc-renilla and incubated in the same manner. At 6 hours later, the cells were washed with PBS, and the cell lysate was collected using Passive Lysis Buffer. This cell lysate was centrifuged at 4°C and 3000 rpm for 1 minute. Thereafter, luciferase activity was evaluated using the supernatant and a Luciferase Assay kit (Promega) with a POWERSCAN·H1 microplate reader (BioTek).

The results are shown in Figure 7. No suppression of expression was observed in a case where TPM3 was not used (bound) as a Staple nucleic acid (Figure 7).

### References

(1) Fire, A.; Xu, S.; Montgomery, M. K.; Kostas, S. A.; Driver, S. E.; Mello, C. C. Nature 1998, 391, 806.
(2) Ambros, V. Nature 2004, 431, 350.
(3) Jackson, A. L.; Linsley, P. S. Nature reviews. Drug discovery 2010, 9, 57.
(4) Jackson, A. L.; Burchard, J.; Leake, D.; Reynolds, A.; Schelter, J.; Guo, J.; Johnson, J. M.; Lim, L.; Karpilow, J.; Nichols, K.; Marshall, W.; Khvorova, A.; Linsley, P. S. Rna 2006, 12, 1197.
(5) Huppert, J. L.; Balasubramanian, S. Nucleic acids research 2007, 35, 406.
(6) Kumari, S.; Bugaut, A.; Huppert, J. L.; Balasubramanian, S. Nature chemical biology 2007, 3, 218.
(7) Katsuda, Y.; Sato, S.; Asano, L.; Morimura, Y.; Furuta, T.; Sugiyama, H.; Hagihara, M.; Uesugi, M. Journal of the American Chemical Society 2016, 138, 9037.
(8) Rothemund, P. W. Nature 2006, 440, 297.
(9) Fujita, H.; Kataoka, Y.; Tobita, S.; Kuwahara, M.; Sugimoto, N. Analytical chemistry 2016, 88, 7137.
(10) Bugaut, A.; Murat, P.; Balasubramanian, S. Journal of the American Chemical Society 2012, 134, 19953.
(11) Hagihara, M.; Yoneda, K.; Yabuuchi, H.; Okuno, Y.; Nakatani, K. Bioorganic & medicinal chemistry letters 2010, 20, 2350.
(12) Jang, S. K.; Davies, M. V.; Kaufman, R. J.; Wimmer, E. Journal of virology 1989, 63, 1651.
Sequence Listing Free Text
SEQ ID NOS: 1 to 68, 71 to 74, 79 to 90: Synthetic DNA
SEQ ID NOS: 69, 70, 75 to 78: Synthetic RNA
## Claims

1. A method for suppressing protein expression, comprising using an oligomer for a target RNA comprising first, second, third, and fourth guanine repeat sequences (referred to as "first G sequence," "second G sequence," "third G sequence," and "fourth G sequence," respectively), the oligomer hybridizing with regions proximal to the guanine repeat sequences, so as to bring the guanine repeat sequences closer together via folding of the oligomer, thereby forming a guanine-quadruplex structure,
wherein a nucleic acid of a part of a region on one end side (one end region) and a nucleic acid of a part of a region on the other end side (other end side region) of the oligomer hybridize with a region that is between the first G sequence and the second G sequence and proximal to both the first G sequence and the second G sequence, a region that is between the second G sequence and the third G sequence and proximal to both the second G sequence and the third G sequence, or a region that is between the third G sequence and the fourth G sequence and proximal to both the third G sequence and the fourth G sequence.

2. The method according to claim 1, wherein the oligomer is designed such that its folding direction faces the guanine quadruplex structure side for hybridization.

3. The method according to claim 1 or 2, wherein the target RNA is mRNA, TPM3, or MYD88.

4. The method according to any one of claims 1 to 3, wherein the oligomer is DNA or RNA.

5. The method according to any one of claims 1 to 4, wherein the oligomer is a dimer.

6. The method according to any one of claims 1 to 5, wherein the protein expression is reverse transcription reaction or protein translation reaction.

7. An oligomer for bringing first, second, third, and fourth guanine repeat sequences (referred to as "first G sequence," "second G sequence," "third G sequence," and "fourth G sequence," respectively) included in a target RNA closer together so as to form a guanine-quadruplex structure,
wherein a nucleic acid of a part of a region on one end side (one end region) and a nucleic acid of a part of a region on the other end side (other end side region) of the oligomer hybridize with a region that is between the first G sequence and the second G sequence and proximal to both the first G sequence and the second G sequence, a region that is between the second G sequence and the third G sequence and proximal to both the second G sequence and the third G sequence, or a region that is between the third G sequence and the fourth G sequence and proximal to both the third G sequence and the fourth G sequence such that the guanine repeat sequences are brought closer together via folding of the oligomer.

8. The oligomer according to claim 7, which is designed such that its folding direction faces the guanine quadruplex structure side for hybridization.

9. The oligomer according to claim 7 or 8, which is DNA or RNA.

10. The oligomer according to any one of claims 7 to 9, which is a dimer.

11. A method for producing the oligomer according to any one of claims 7 to 10, comprising designing and synthesizing the oligomer such that
a nucleic acid of a part of a region on one end side (one end region) and a nucleic acid of a part of a region on the other end side (other end side region) of the oligomer hybridize with a region that is between the first G sequence and the second G sequence and proximal to both the first G sequence and the second G sequence, a region that is between the second G sequence and the third G sequence and proximal to both the second G sequence and the third G sequence, or a region that is between the third G sequence and the fourth G sequence and proximal to both the third G sequence and the fourth G sequence.

12. A kit for suppressing protein expression, comprising the oligomer according to any one of claims 7 to 10 or an oligomer produced by the method according to claim 11.
